# EUROPÄISCHE PATENTSCHRIFT

(11) **EP 1 925 665 B1**
(45) Veröffentlichungstag und Bekanntmachung des Hinweises auf die Patenterteilung: **05.08.2009**
(21) Anmeldenummer: 07113519.8
(22) Anmeldetag: 23.08.2002
(51) Int. Cl.: C12N 15/11, A61P 17/10, A61K 31/713

(54) **Verwendung von molekularbiologisch hergestellten, nicht-viralen Wirkstoffen zur Behandlung der Akne**
Use of non-viral active agents manufactured with a molecular-biological method for treating acne
Utilisation d'agents actifs non viraux biologiques moléculaires destinés au traitement de l'acné

(30) Priorität: 23.08.2001 DE 10141443
(43) Veröffentlichungstag der Anmeldung: 28.05.2008
(62) Teilanmeldung aus: 02796268.7
(73) Patentinhaber: RiNA Netzwerk RNA-Technologien GmbH, 14195 Berlin (DE); Zouboulis, Christos C., 14129 Berlin (DE)
(72) Erfinder: Zouboulis, Christos C., 14129 Berlin (DE)
(74) Vertreter: Jungblut, Bernhard Jakob

(56) Entgegenhaltungen:
- WO-A-95/07924
- WO-A-96/02256
- HARTWELL JOHN ET AL: "Antisense oligonucleotides: A novel approach for the treatment of androgenic skin disorders." JOURNAL OF INVESTIGATIVE DERMATOLOGY, Bd. 108, Nr. 4, 1997, Seite 653, XP008021025 Annual Meeting of the Society for Investigative Dermatology;Washington, D.C., USA; April 23-27, 1997 ISSN: 0022-202X
- BHAN PURSHOTAM ET AL: "Inhibition of 5-alpha-reductase (type-II) expression by antisense 3'-deoxy-(2'-5')oligonucleotide chimeras." NUCLEOSIDES & NUCLEOTIDES, Bd. 16, Nr. 7-9, Juli 1997 (1997-07), Seiten 1195-1199, XP001149287 ISSN: 0732-8311

## Beschreibung

Die vorliegende Erfindung betrifft die Verwendung von molekularbiologisch hergestellten, nicht-viralen Wirkstoffen (Antisense-Oligonukleotide, Ribozyme, stabilisierte Ribozyme, Aptamere, Spiegelaptamere, chimerische RNA/DNA-Oligonukleotide, nackte Plasmid-DNA, liposomal verkapselte DNA) zur Behandlung der Akne und der akneiformen Dermatosen, inkl. der Rosazea. Die Wirkstoffe können in einer pharmazeutischen Zusammensetzung nach einem beliebigen herkömmlichen Applikationsweg verabreicht werden.

Es wird angenommen, dass Akne-Läsionen aufgrund der Interaktion von miteinander in Beziehung stehenden, komplexen Vorgängen entstehen: einer erhöhten Produktion und Exkretion des Sebums (Cunliffe WJ, Shuster S: Pathogenesis of acne. Lancet. 1969; 1(7597): 685-687), einer duktalen Kornifizierung (Holmes RL, Williams M, Cunliffe WJ: Pilosebaceous duct obstruction and acne. Br J Dermatol. 1972; 87: 327-332 und 35), einer abnormalen Zahl und Funktion von *Propionibacterium acnes* (Cunliffe WJ, Clayden AD, Gould D, Simpson NB: Acne vulgaris-its aetiology and treatment. A review. Clin Exp Dermatol. 1981; 6: 461-469) sowie die Produktion entzündlicher Mediatoren, die zur Bildung von Papeln, Pusteln und zeitweise tiefen entzündlichen Läsionen führen.

Die erhöhte Produktion und Exkretion des Sebums bei der Akne wird unter anderem durch eine Beschleunigung des Differenzierungszyklus der Talgdrüsenzellen (Sebozyten) verursacht. Die Androgene sind die wichtigsten Regulatoren der Sebozyten, sowohl in der ontogenetischen Entwicklung als auch in der Kontrolle der Aktivität der differenzierten Sebozyten, sie verstärken Lipogenese, Proliferation und terminale Differenzierung von Sebozyten sowie einiger Keratinozytenpopulationen in der Haut. Die Regulation erfolgt durch die Bindung eines Androgen-Androgenrezeptorkomplexes an kompetente Stellen der genomischen DNA, wodurch die Synthese von spezifischen Proteinen initiiert wird. Testosteron und 5α-Dihydrotestosteron (DHT) sind dabei die beiden wichtigsten Vertreter der Androgene; wobei sich DHT mit deutlich höherer Affinität als Testosteron an den Androgenrezeptor bindet. DHT wird intrazellulär aus dem Testosteron mit Hilfe des Enzyms 5α-Reduktase synthetisiert. Diese metabolische Funktion findet bei der Haut insbesondere in den Sebozyten statt, wo kürzlich eine überproportionale 5α-Reduktase-Aktivität lokalisiert wurde. Es wurden zwei Gene gefunden, die für zwei verschiedene Isoenzyme (Typ-1 und Typ-2) des 5α-Reduktase-Enzyms codieren (Genbank locus: HUM5AR und HUMSRDA), wobei das Isoenzym-Typ-1 besonders in den Talgdrüsen aktiv ist.

Darüber hinaus wurde die höchste Zahl der intrazellulären Androgenrezeptoren der Haut in den Sebozyten nachgewiesen. Aus diesen Gründen wird der Hemmung der 5α-Reduktase-Aktivität (besonders des Typ-1-Isoenzyms) und des Androgenrezeptors in der Haut eine große Bedeutung für die Hemmung der terminalen Differenzierung der Sebozyten und somit für die Behandlung der Seborrhoe und der Akne zugesprochen. Erste experimentelle und klinische Ergebnisse bei Anwendung spezifischer Hemmer des 5α-Reduktase-Isoenzyms-Typ-1 und des Androgenrezeptors bestätigen diese Annahme, allerdings ist dafür die systemische Gabe der entsprechenden Wirkstoffe notwendig, die wegen ihrer starken Auswirkungen auf den gesamten Körper unerwünscht ist.

Seit kurzer Zeit werden Versuche unternommen, eine gezielte Therapie verschiedener Erkrankungen mittels molekularbiologisch hergestellter Wirkstoffe zu unternehmen. Zum Beispiel lassen sich mit der Antisense-Technologie neuartige Medikamente entwickeln. Traditionelle Xenobiotika wirken auf Proteine, die im Organismus zu einer Krankheit geführt haben. Antisense-Moleküle arbeiten auf genetischer Ebene, indem sie spezifisch die Translation des Proteins blockieren, welches eine Störung verursacht. Daher haben Medikamente auf Antisensebasis das Potential selektiver, und so effektiver und weniger toxisch zu wirken. In den letzten Jahren wurde die Entwicklung von Effektormolekülen auf Nukleinsäurebasis, den sogenannten Aptameren, erfolgreich zur Hemmung bzw. Aktivierung verschiedener Enzyme und Rezeptoren eingesetzt [Ruckman J. et al.: 2'-Fluoropyrimidine RNA-based aptamers to the 165-amino acid form of vascular endothelial growth factor (VEGF165). Inhibition of receptor binding and VEGF-induced vascular permeability through interactions requiring the exon 7-encoded domain. J. Biol. Chem. 1998; 273: 20556-20567].

Zur Zeit laufen 15 klinische Studien, in denen Antisense-Oligonukleotide eingesetzt werden. Die systemische Behandlung mit Antisense-Oligonukleotiden ist wegen der potentiellen Zytotoxizität der Antisense-Oligonukleotide und der Trägermoleküle wie kationische Lipide noch problematisch. Die Behandlung von pathologischen Zuständen, die die Haut beeinträchtigen, können durch lokale Applikation teilweise verbessert werden. Jedoch ist der Erfolg der Antisense-Strategie bisher durch die geringe Aufnahmefähigkeit der Oligonukleotide in den Zielzellen, insbesondere durch die geringe Aufnahmefähigkeit des Transfektionsreagenz und die unzureichende intrazelluläre Kompartimentierung beschränkt. Menschliche Epithelzellen, insbesondere solche der Haut, sind das bevorzugte Zielgewebe, um die Aufnahmefähigkeit und die Wirkung von Antisense-Oligonukleotiden für therapeutische und diagnostische Zwecke *in vitro* und *in vivo* zu beurteilen.

Der biologische Effekt der Antisense-Oligonukleotide wird dabei durch verschiedene Faktoren beeinflusst, wie die lokale Konzentration des Oligonukleotids, die initiale Konzentration des Oligonukleotids, die lokale Konzentration des Oligonukleotids am Zielgewebe, die Penetration des Oligonukleotids durch die Zellmembran, das Lipid/Oligonukleotid-Verhältnis und die daraus resultierende elektrische Ladung, die Abbaugeschwindigkeit des Oligonukleotids und die DNA- bzw. RNA-Verlängerungsrate des Zielgens. Molekularbiologisch hergestellte Medikamente auf Antisense-, Ribozym-, stabilisierte Ribozym-, Aptamer-, Spiegelaptamer-, chimerische RNA/DNA-Oligonukleotid-, nackte Plasmid-DNA- und liposomal verkapselte DNA-Basis haben so das Potential, selektiver und so effektiver und weniger toxisch zu wirken als herkömmliche Therapeutika.

Die Entwicklung von Antisense-Oligonukleotiden mit Spezifizität für Zielsequenzen von Genen, die für den Androgenrezeptor und 5α-Reduktase kodieren, ist aus dem US-Patent Nr. 5,877,160 bekannt. Durch verringerte Produktion der Transkriptionsprodukte konnte die Progression der männlichen Glatzenbildung gehemmt werden. Durch Verabreichung speziell synthetisierter Antisense-Oligonukleotide (Oligoribonukleotide und Phosphorothioat-Oligonukleotide) gegen den Androgenrezeptor und 5α-Reduktase konnte die Konzentration von an den Androgenrezeptor gebundenem 5α-Dihydrotestosteron (DHT) in Haarfollikeln merklich verringert werden, ohne die Testosteron-Synthese und/oder dessen Metabolismus in anderen Geweben zu beeinflussen. Die Androgen-Bindungskapazität für DHT in Hautzellen nach Oligonukleotidapplikation war wesentlich reduziert.

Die Aufgabe der Erfindung besteht somit darin, die Therapiemöglichkeiten gegen Akne zu erweitern und dabei die Wirkung unter gleichzeitiger Reduzierung von Nebenwirkungen möglichst zu verbessern, ohne die Nachteile infolge systemischer Gabe in Kauf zu nehmen.

Die Aufgabe wird erfindungsgemäß gelöst durch Verwendung von einem oder mehreren molekularbiologisch hergestellten, nicht-viralen Wirkstoffen, insbesondere Antisense-Oligonukleotiden mit Spezifizität für Zielsequenzen in Genen, die für den Androgenrezeptor und/oder die 5α-Reduktase kodieren, zur Behandlung von Akne. Außer den insbesondere bevorzugten Antisense-Oligonukleotiden sind ebenfalls Ribozyme, stabilisierte Ribozyme, Aptamere, Spiegelaptamere, chimerische RNA/DNA-Oligonukleotide, nackte Plasinid-DNA, oder liposomal verkapselte DNA einsetzbar.

Gemäß dem Wirkungskonzepts einer insbesondere bevorzugten Ausführungsform der Erfindung wurden Antisense-Oligonukleotide ausgewählt und konstruiert, die komplementär zu einer RNA-Sequenz sind, die aus einem Zielgen in eine einzelsträngige DNA-Zielsequenz oder eine Einzelstrang-RNA- oder DNA innerhalb eines Duplexmoleküls transkribiert wird. Solche Antisense-Oligonukleotide umfassen vorzugsweise wenigstens eine der folgenden Sequenzen:
*5'-CTG CAC TTC CAT CCT TGA GCT TGG C-3'*
   *ein Phosphorothioat-Antisense-Oligonukleotid (Anti-DNA) und*
*5 ' -GCC AAG CUC AAG GAU GGA AGU GCA G-3 '*
   *ein Methylribosyl-Antisense-Oligonukleotid (Anti-RNA),*
die den Nukleotidpositionen des Androgenrezeptorgens 13 zu 12 entsprechen (Transkriptions-Initialposition).

Die so konstruierten Duplexmoleküle hemmen die Translation, die Prozessierung oder den Transport einer mRNA-Sequenz oder führen zu Digestion durch das Enzym Ribonuklease-H. Ribonuklease-H degradiert den RNA-Strang eines RNA-DNA-Duplexmoleküls.

Die Oligonukleotide interagieren mit anderen Biomolekülen, wie Lipiden, Kohlenhydraten und Proteinen. Sie induzieren Non-Antisense-Effekte, die inkorrekterweise, als Antisense-Effekte interpretiert werden können. Um die Spezifizität unserer Antisense-Oligonukleotide gegen den Androgenrezeptor zu sichern, haben wir auch Mismatch-Oligonukleotide, wie folgt, konstruiert:
*5'-CTG CCC TTC AAT CCC TGA GTT TGG C-3'*

Ebenso können degenerierte Sequenzen oder ähnliche Sequenzen mit vergleichbarer Spezifizität für die beabsichtigten Zielsequenzen eingesetzt werden.

Weiter bevorzugt besitzen die erfindungsgemäß verwendeten Antisense-Oligonukleotide Spezifizität für Epithelzellen, insbesondere humane Epithelzellen. Besonders geeignet ist die erfindungsgemäße Verwendung in Sebozyten und epidermalen Keratinozyten. In einem weiteren Gegenstand der Erfindung wird eines oder mehrere der Antisense-Oligonukleotide, Ribozyme, stabilisierte Ribozyme, Aptamere, Spiegelaptamere, chimerische RNA/DNA-Oligonukleotide, nackte Plasmid-DNA, oder liposomal verkapselte DNA in einer geeigneten pharmazeutischen Zusammensetzung mit üblichen pharmazeutisch akzeptablen Trägern und ggf. weiteren Hilfsstoffen eingesetzt. Besonders geeignet ist die Verabreichung in Form einer lipidvermittelten Transfektion von Sebozyten und/oder Keratinozyten. Insbesondere bevorzugt ist hierzu die Aufnahme der Antisense-Oligonukleotide mittels einer Liposomen-vermittelten, speziell kationischen Liposomen-vermittelten Transfektion des Zielgewebes.

Im Rahmen der vorliegenden Erfindung wurde gefunden, dass mit den erfindungsgemäß verwendeten Wirkstoffen, insbesondere den Antisense-Oligonukleotiden die androgenerge Stimulation der Talgdrüsenaktivität wirkungsvoll inhibiert werden kann und daher molekularbiologisch hergestellte Moleküle (Antisense-Oligonukleotide, Ribozyme, stabilisierte Ribozyme, Aptamere, Spiegelaptamere, chimerische RNA/DNA-Oligonukleotide, nackte Plasmid-DNA, oder liposomal verkapselte DNA) als ausgezeichnete Wirkstoffe zur Behandlung der Akne eingesetzt werden können.

Testosteron ist das wesentliche zirkulierende Androgen im Körper, besitzt aber geringe Aktivität in Hautzellen. Ein viel aktivierer Metabolit ist 5α-Dihydrotestosteron (DHT) . DHT wird nach Reduktion von Testosteron durch das Enzym 5α-Reduktase gebildet. Die Effekte von Androgenen werden durch Binden des Liganden (hauptsächlich DHT) an den Androgenrezeptor vermittelt, welcher entweder im Zellkern vorliegt oder an das Zytoplasma transferiert wird. Der Androgenrezeptor gehört zu der Subfamilie von nukleären Rezeptoren, welche Kortikosteroid-, Thyroidhormon-, Retinsäure-, Vitamin D-, Estrogen-, Progesteron- und PPA-Rezeptoren beinhaltet, deren Aktivität durch die enge und spezifische Bindung des Liganden gesteuert wird.

Gemäß dieser Erfindung wurde gefunden, dass die biologische Aktivität des Androgenrezeptors und der 5α-Reduktase in nativen und in mit Androgenrezeptor-Antisense-transfizierten Sebozyten bzw. Keratinozyten nach ihrer Stimulation mit Androgenen inhibiert werden kann. Die transiente Reduktion der Androgenrezeptor-Expression sowie der Expression von 5α-Reduktase nach transienter Transfektion von Hautzellen wie Sebozyten und Keratinozyten zeigt, dass die erfindungsgemäße Verwendung der Antisense-Oligonukleotide ein hochwirksames Mittel zur therapeutischen Behandlung von Akne und von akneiformen Dermatosen, inkl. der Rosazea ist.

Es wird angenommen, dass die überraschende Effektivität der erfindungsgemäß verwendeten Antisense-Oligonukleotide gegenüber dem Androgenrezeptor und der 5α-Reduktase zurückzuführen ist auf die spezifische Bindung an eine vom Zielgen transkribierte RNA-Sequenz und Bildung eines DuplexStranges. Dieser Duplex-Strang inhibiert die Translation, die Prozessierung und den Transport der mRNA-Sequenz oder führt zu einem Verdau durch Ribonuklease-H. Die erfindungsgemäß verwendeten Oligonukleotide können die Ribonuklease-H aktivieren. Die spezifische Bindung der erfindungsgemäß verwendeten Antisense-Oligonukleotide kann auch an doppelsträngiger Nukleinsäure unter Bildung eines Triplex-Komplexes erfolgen.

Nachfolgend wird die vorliegende Erfindung unter Bezugnahme auf bevorzugte Ausführungsformen im Zusammenhang mit den begleitenden Figuren genau beschrieben. In den Figuren ist:

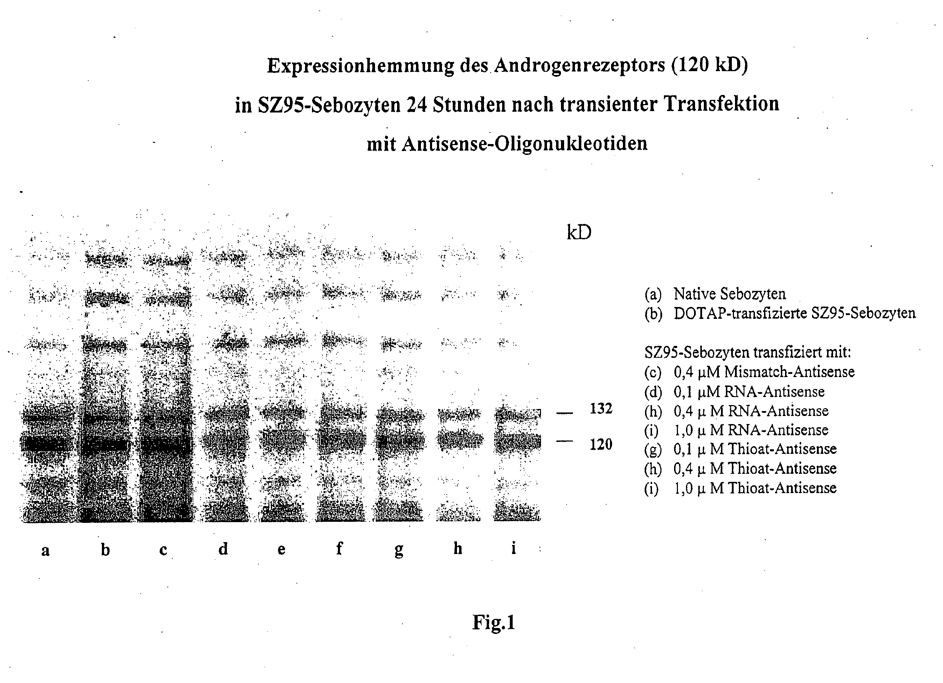

Fig. 1 eine erläuternde Darstellung der Expressionshemmung des Androgenrezeptors (120 kD) in SZ95-Sebozyten 24 Stunden nach transienter Transfektion mit Antisense-Oligonukleotiden. Die Figur zeigt Westernblots, in denen das entsprechende aufgetrennte Protein mit polyklonalem Anti-Androgenrezeptor-Antikörper aus dem Hasen und mit gegen den Hasen gerichteten Immunoglobulin G, das mit sekundärer Meerrettichperoxidase konjugiert war, inkubiert wurde. Die Blots wurden durch Chemolumineszenz ausgewertet und die Intensitäten der Androgenrezeptor-Proteinbanden wurden densitometrisch mit dem Computerprogramm TINA 2.0 bestimmt.
Fig. 2 eine Erläuterung der Expressionshemmung des Androgenrezeptors (120 kD) in SZ95-Sebozyten 14 Stunden nach transienter Transfektion mit Antisense-Oligonukleotiden mit dem oben beschriebenen Detektionssystem, und
Fig. 3 eine Erläuterung der Expressionshemmung des Androgenrezeptors (120 kD) in epidermalen Keratinozyten 17 Stunden nach transienter Transfektion mit Antisense-Oligonukleotiden mit dem oben beschriebenen Detektionssystem,
Fig. 4 eine Übersicht der Testosteron-stimulierten Proliferation von nativen SZ95-Sebozyten nach einer Stimulationszeit von 72 Stunden bei unterschiedlichen Konzentrationen von Testosteron,
Fig. 5 eine Übersicht der DHT-stimulierten Proliferation nativer SZ95-Sebozyten nach einer Stimulationszeit von 72 Stunden bei unterschiedlichen Konzentrationen von DHT,
Fig. 6 eine Übersicht der Proliferationsraten von mit DOTAP behandelten SZ95-Sebozyten nach einer Stimulationszeit von 72 Stunden,
Fig. 7 eine Übersicht der Inhibition der Testosteroninduzierten Stimulation der SZ95-Sebozyten-Proliferation durch Thio-Antisense-Oligonukleotide bei verschiedenen Konzentrationen von Testosteron bzw. DHT nach einer Stimulationsdauer von 44 Stunden, und
Fig. 8 eine Übersicht der Inhibition der Testosteroninduzierten Stimulation der SZ95-Sebozyten-Proliferation durch RNA-Antisense-Oligonukleotide bei verschiedenen Konzentrationen von Testosteron bzw. DHT nach einer Stimulationsdauer von 44 Stunden.

Die erfindungsgemäß verwendeten Oligonukleotide können einen geladenen oder ungeladenen Strang aufweisen. Neutrale Oligomere sind bevorzugt, da sie von der Haut leicht aufgenommen werden, wenn sie topisch appliziert werden. Sie werden schnell aus dem Plasma freigesetzt und in den Urin abgegeben. Die erfindungsgemäß verwendeten Oligonukleotide können ferner bevorzugt chemisch modifiziert sein. Insbesondere bevorzugt sind neutrale Methylphosphonatoligomere, die lediglich Methylphosphonat-Internukleosidyl-Bindungen aufweisen. Die Gegenwart von Methylphosphonat oder anderen neutralen Internukleotid-Bindungen in dem Oligomer stellen eine exonukleare Resistenz bereit. Die Verwendung von Nukleosideinheiten mit 2'-O-Alkyl- oder 2'-Halo- und insbesondere 2'-O-Fluoro- oder 2'-Methyl-Ribosyleinheiten in den neutralen Oligomeren können in vorteilhafter Weise die Hybridisierung des Oligomers mit dessen komplementärer Zielsequenz verbessern. Weitere bevorzugte Oligomere beinhalten Phosphorothioat-Oligonukleotide. Derartige Oligonukleotide besitzen eine längere Halbwertszeit *in vivo,* da ihre neutrale Struktur die Rate des Nukleaseabbaus reduziert, während die spaltende oder vernetzende Einheit die Inaktivierung der Polynukleotid-Zielsequenzen fördern kann.

Eine besonders wirksame Maßnahme, die erfindungsgemäßen Oligonukleotide in die Epithelzielzellen einzubringen, ist die liposomale Transfektion. Durch diese Maßnahme wird die Aufnahme der Antisense-Moleküle in die Zellen erhöht. Die Mischung von liposomalen Transfektionsreagenzien mit den Oligonukleotiden führt zu stabilen Komplexen, die direkt dem Zellkulturmedium zugegeben werden können. Diese Komplexe binden sich an der Zelloberfläche, fusionieren mit der Zellmembran und entlassen die negativ geladenen Oligonukleotide in das Zytoplasma. Besonders wirksam ist die Transfektion unter Verwendung von kationischen Lipiden, die eine erhöhte Transfektionsrate der Antisense-Oligonukleotide bewirken können. Bei der Auswahl des Transfektionsreagenz ist es insbesondere wichtig, eine nicht-toxische Aufnahme der Oligonukleotide in den kultivierten Epithelzellen, insbesondere Sebozyten und Keratinozyten zu bewirken.

Gemäß einer insbesondere bevorzugten Ausführungsform der Erfindung werden polykationische Lipide, insbesondere das Polykation DOTAP zur Transfektion von SZ95-Sebozyten und insbesondere das Polykation L-Ornithin zur Transfektion von Keratinozyten verwendet. Unter Einsatz dieser Lipide werden effektive Transfektionssysteme ohne wesentliche Zytotoxizität bereitgestellt. Die herabgesetzte Zytotoxizität kann durch Variation der Kulturbedingungen wie der Konfluenz, der Oligonukleotid-Konzentration und des Ladungsverhältnisses zwischen Lipid und Oligonukleotid gesteuert werden. Gewünscht ist auch eine Einstellung des Verhältnisses von Lipid zu Oligonukleotid. Eine ausreichende Lipidmenge ist zur Erzeugung von Komplexen mit dem Oligonukleotid erforderlich, um eine positive Gesamtladung zu schaffen. Es wird angenommen, dass die positive Ladung des LipidOligonukleotid-Komplexes die Interaktion mit der negativ geladenen Zelloberfläche erleichtert. Ein Überschuss von Lipid erhöht jedoch wiederum die Toxizität gegenüber den Zellen und führt so zu einer Veränderung der Zellmorphologie, einem verringerten Wachstum und schließlich zum Zelltod. Für SZ95-Sebozyten hat sich ein Ladungsverhältnis von 2:1 von DOTAP und für Keratinozyten ein Ladungsverhältnis von etwa 1:1 von L-Ornithin als besonders geeignet erwiesen.

Die Verabreichung des Wirkstoffs kann über übliche Applikationswege erfolgen, z.B. auf systemische Art und Weise, enteral, insbesondere oral oder rektal, transdermal, nasal mittels Inhalation sowie parenteral, insbesondere mittels Injektion (subkutan, intramuskulär oder intravenös), oder dergleichen. Behandelt werden können Menschen und Tiere wie beispielsweise Säugetiere und Nager. Damit die systemische Beeinträchtigung durch den Wirkstoffeinsatz möglichst gering gehalten bzw. ausgeschlossen wird, ist die lokale und insbesondere die topische Applikation bevorzugt. Hierfür können trägerfreie Systeme (in Salben, Cremes, Gelen, Ölen, Emulsionen, Lotionen, Pasten, oder Lösungen, bzw. anderen Zusammensetzungen) oder auf Trägern wie Pflastern, Verbandsmaterial etc. basierende Systeme angewandt werden. Eine besonders bevorzugte Einsatzmöglichkeit besteht in der Applikation der Oligonukleotid-Zubereitung in Liposomen oder in Liposomen, die in eine andere Zusammensetzung aufgenommen werden.

Die Antisense-Oligonukleotide können in pharmazeutischen Zusammensetzungen zum Einsatz kommen, die eine gegen Akne wirksame Inhibierung der Translation des Androgenrezeptors und/oder der 5α-Reduktase hervorruft, ggfs. in Kombination mit anderen Anti-Akne-Mitteln, in Verbindung bzw. Beimengung mit für die jeweiligen oben bezeichneten Applikationsarten üblichen, pharmazeutisch akzeptablen Arzneimittelträgern umfassen.

Geeignet sind beispielsweise Tabletten oder Kapseln mit dem bzw. den wirksamen Bestandteil(en) zusammen mit Streckmitteln wie z.B. Lactose, Dextrose, Sucrose, Manitol, Sorbitol, Cellulose und/oder Glycin, Gleitmitteln wie z.B. Siliciumdioxid, Talg, Stearinsäure sowie dessen Magnesium- oder Calciumsalze und/oder Polyethylenglycol, für Tabletten ferner Bindemittel wie z.B. Magnesiumaluminiumsilikat, Stärke, Gelatine, Tragakant, Methylcellulose, Natriumcarboxymethylcellulose und/oder Polyvinylpyrrolidon, sowie, falls gewünscht, Desintegriermittel wie z.B. Stärke bzw. modifizierte Stärke, Agar, Algeininsäure bzw. dessen Natriumsalz, oder Mischungen davon, und ggf. Absorbentien, Färbemittel, Geschmacksstoffe und Süßstoffe. Injektionsfähige Zusammensetzungen sind beispielsweise wässrige isotonische Lösungen oder Suspensionen, und Suppositorien werden vorzugsweise aus Fettemulsionen oder -Suspensionen hergestellt. Lokale bzw. topische pharmazeutische Zusammensetzungen sind beispielsweise Salben, Cremes, Gele, Öle, Emulsionen, Lotionen, Pasten, Liposomen oder Lösungen und enthalten neben den Wirkstoffen die für die genannten Formulierungen entsprechenden Zusatzstoffe. Bei lokal topischen Applikationen können ferner Mittel zur Steigerung der perkutanen Resorption zugesetzt werden, beispielsweise Hyaluronidate, Dimethylsulfoxid (DMSO) und dergleichen. Die genannten pharmazeutischen Zusammensetzungen können sterilisiert sein und/oder weitere Hilfsstoffe enthalten, wie Konservierungsmittel, Stabilisatoren, Benetzungsmittel, Emulgatoren usw. Die Zusammensetzungen können mittels herkömmlicher Verfahren zum Mischen, Granulieren oder Beschichten hergestellt werden. Der bzw. die aktiven Wirkstoffe können in unterschiedlichen Zusammensetzungen und Mengen von 0,001 bis 50 Gew.-%, vorzugsweise 0,01 bis 40 Gew.-%, weiter bevorzugt bis 20 Gew.-% und insbesondere bis 10 Gew.-% bezogen auf das Gesamtgewicht der pharmazeutischen Zusammensetzungen, enthalten sein.

Die vorliegende Erfindung wird nachfolgend anhand der folgenden Beispiele näher erläutert.

### Beispiel 1

### Behandlung von Sebozyten und Keratinozyten mit Transfektionsreagenzien in vitro

Immortalisierte humane Sebozyten (SZ95-Sebozyten) wurden in einem modifizierten DMEM/Ham's F-12 (1:1) Medium mit 10% (v/v) fötalem Rinderserum (FCS) and 5 ng/ml rekombinantem, humanem epidermalem Wachstumsfaktor (Biochrom, Berlin, Deutschland) mit 5% CO₂ und 37°C gehalten und bei Passagen 50-55 eingesetzt.

Primäre humane epidermale Keratinozyten aus neonatalem Vorhautgewebe wurden in serumfreiem Keratinozyten-Medium (Gibco BRL, Berlin, Germany), ergänzt durch 5 ng/ml rekombinanten humanen epidermalen Wachstumsfaktor und 50 µg Rinderhypophysen-Extract kultiviert. Die Keratinozyten wurden zwischen Passagen 2-5 transfiziert.

Sowohl die Transfektionsreagenzien als auch die Oligonukleotide können die Vitalität der Sebozyten bzw. Keratinozyten beeinflussen. Daher wurden im ersten Schritt die Zellkulturen mit verschiedenen Transfektionsreagenzien in Abwesenheit von Oligonukleotiden inkubiert, um ihre Zytotoxizität zu testen:
1. Tfx-10
2. Tfx-20 (Promega)
3. Tfx-30

Die Tfx-Reagenzien sind eine Mixtur eines synthetischen, kationischen Lipidmoleküls [N,N,N',N'-Tetramethyl-N,N'-bis(2-hydroxyethyl)-2,3-di(oleoyloxy)-1,4-butandiammoniumiodid] und L-Dioleoylphosphatidylethanolamin (DOPE). Alle Tfx-Reagenzien enthalten dieselbe Konzentration der kationischen Lipidkomponente, aber mit verschiedenen molaren Verhältnissen des fusogenen Lipids DOPE.
4. DAC-30 (Eurogentec)
   DAC-30 ist die liposomale Form eines monokationischen Cholesterolderivates.
5. DOTAP (Boehringer)
   DOTAP ist eine liposomale Form des kationischen Lipids N-[1-(2,3-dioleoyloxy)propyl]-N,N,N-trimethylammoniummethylsulfat (DOTAP).
6. Effectene (QIAGEN)
7. SuperFect
8. PerFect Lipids (Invitrogen)
   Pfx-1 bis Pfx-8 sind acht verschiedene kationische Lipide, die sich in ihrem Verhältnis positive Ladung pro molekulare Lipideinheit unterscheiden.
9. Poly-L-Ornithin
10. Polybrene (Sigma)

Die Transfektionsansätze werden mit Sebozyten- bzw. Keratinozytenkulturen durchgeführt, die nicht mehr als 80 %, bevorzugt zu 50 - 70% konfluent sind. Der zytotoxische Effekt der verschiedenen Transfektionsreagenzien wurde mit Hilfe eines LDH-Assays bestimmt. Dieser kolorimetrische Test zur Quantifizierung von Zelltod und -Lysis basiert auf der Messung von Laktatdehydrogenase-Aktivität, die vom Zytosol der geschädigten Zellen in den Medienüberstand abgegeben wird.

Es wurden folgende Parameter variiert, um die Transfektionseffizienz zu optimieren:

**a) Serumkonzentration:** Das Sebozytenmedium (modifiziertes DMEM/HAM's F-12) wird mit verschiedenen Serumkonzentrationen (5%, 2%, serumfrei) supplementiert. Für eine optimale Transfektion mit DOTAP war serumfreies Medium erforderlich. Die Toxizitätsrate verdoppelte sich bei einer Transfektion mit DAC-30 (4 µg/ml) in Gegenwart von 5% fötalem Kälberserum. DOTAP-Vesikel zeigten einen moderaten zytotoxischen Effekt verglichen mit den anderen getesteten Transfektionsreagenzien wie die Tfx-Kationen. Die mit Fluoreszenzmikroskopie überprüfte Transfektionseffizienz war in serumhaltigem bzw. serumfreiem Medium äquivalent. Der Hypophysenextrakt, mit dem das Keratinozytenmedium supplementiert wird, hatte keinen Einfluß auf die Transfektion der primären Keratinozyten mit pFx-5 oder mit Poly-L-Ornithin. Allerdings wurde wegen der besseren Vergleichbarkeit von Sebozyten und Keratinozyten auch die Keratinozytentransfektion in hypophysenfreiem Medium durchgeführt.

b) Verhältnis der Ladungen von Lipid (L) zum Oligonukleotid (O)

| Ladungsverhältnis L/O | Transfektionsreagenz µg/ml] | | | | | |
|---|---|---|---|---|---|---|
| | Tfx | -10 | -20 | 50 | DAC-30 | DOTAP |
| 2:1 | | 26 | 13 | 6 | 2 | 2 |
| 3:1 | | 36 | 19 | 8 | 4 | 5 |
| 4:1 | | 51 | 27 | 12 | 8 | 10 |

Das Ladungsverhältnis des Transfektionsreagenz zeigte sich als kritischer Parameter; der Anteil der positiven Ladung der kationischen Lipidkomponente sollte daher größer sein als der negative Ladungsanteil der Antisense-Oligonukleotide. Als Kontrolloligonukleotide wurden Random-Primer (ATCG)₅ eingesetzt. Niedrige Konzentrationen von DAC-30 (4 µg/ml) und DOTAP (2 µg/ml) in serumfreiem Medium und in einem Ladungsverhältnis von 2:1 bzw. 3:1 zeigten keine zytotoxische Wirkung. Höhere Mengen von Tfx-10 und Tfx-50 waren ebenfalls nicht letal, allerdings war mit den Tfx-Reagentien kein ausreichender Einbau der Random-Oligonukleotide in die Sebozyten nachzuweisen. Die Effizienz der Keratinozytentransfektion mit pFx-5 konnte durch Steuerung der Oligonukleotidkonzentration und der Lipidmenge vorteilhaft gesteigert werden. Mit abnehmender Randomkonzentration von 1 µM zu 0,5 µM in einem pFx-5 Verhältnis von 1:1 konnte der zytotoxische Effekt minimiert werden.

c) Transfektionszeit (1 h/4 h/24 h/36 h): Die Transfektionszeiten variierten, abhängig vom Zelltyp und vom Transfektionsreagenz. Bei den Sebozyten konnte die Transfektionszeit mit DOTAP von einer bis auf 36 Stunden ausgedehnt werden. Die optimale Transfektionsdauer mit DOTAP kombiniert mit (ATCG)₅-Random-Primern lag bei 4 Stunden. Im Vergleich dazu war das Risiko einer Zellschädigung bei DAC-30-mal höher und nahm bei einer Transfektion mit Tfx-10 nach einer längeren Inkubationszeit als 4 Stunden signifikant zu.
Bei den primären Keratinozyten ließ sich eine Transfektion nur mit DAC-30, Pfx-5 und Poly-L-Ornithin durchführen, alle anderen Transfektionsreagenzien erwiesen sich bereits nach einer zweistündigen Inkubation als toxisch. Die besten Resultate ließen sich mit dem Polykation-L-Ornithin 12 µg/ml Transfektionsmedium und anschließendem DMSO-Schock (25% DMSO für 4 Minuten bei Raumtemperatur) bzw. mit DAC-30 im Ladungsverhältnis von 2:1 (3:1) erreichen. Die Transfektionszeit konnte auf 24 Stunden ausgedehnt werden, ohne daß die Zytotoxizität anstieg. Polybrene (30 µg/ml) induzierte eine höhere Zellschädigungsrate.

### Überprüfung der Transfektionseffizienz durch Fluoreszenzmikrokopie

Der Nachweis der zellulären Aufnahme der Antisense-Oligonukleotide erfolgt durch Detektion von Fluoreszenz im Zytoplasma oder Zellkern. Die Zellen wurden für 24 Stunden mit FITC-(ATCG)₅ unter den jeweils optimalen Transfektionsbedingungen inkubiert. Die Sebozyten ließen sich am besten mit DOTAP (Ladungsverhältnis 2:1) transfizieren. Die Keratinozyten wiesen einen guten FITC-Einbau mit Poly-L-Ornithin auf. In beiden Zelltypen nahm die Transfektionseffizienz proportional mit der Transfektionszeit zu. Die zelluläre Fluoreszenz wurde stärker und die Random-Oligonukleotide konnten sowohl innerhalb des Zellkerns (bei den Sebozyten) als auch im Zytoplasma (bei den Keratinozyten) nachgewiesen werden.

Vor der transienten Transfektion der Sebozyten bzw. Keratinozyten mit spezifischen Antisense-Oligonukleotiden, wurde die Expression beider Zielmoleküle - 5α-Reduktase und Androgenrezeptor - in nativen SZ95-Sebozyten und Keratinozyten untersucht.

### Reverse Transkriptase-Polymerasekettenreaktion (RT-PCR)

Der RNA-Nachweis erfolgte mittels RT-PCR unter semiquantitativen Bedingungen (Skalierung der mRNA-Proben mit β-Aktin). Vollständige RNA wurde aus Zellen mit einem Gesamt-RNA Isolationssystem (Qiagen, Hilden, Deutschland) isoliert. Nach Denaturierung (65°C, 5 min), wurden 5 µg RNA in cDNA mit einem gebrauchsfertigen T-geprimten First-Strand Kit (Amersham Pharmacia, Freiburg, Germany) transkribiert. cDNA-Fragmente des Androgenrezeptors and von β-Aktin wurden mit den folgenden Primern amplifiziert: β-Aktin in Leserichtung (5'-AGC CTC GCC TTT GCC GA-3'), reverse Richtung (5'-CTG GTG CCT GGG GCG-3'); Androgenrezeptor in Leserichtung (5'-GAA GAC CTG CCT GAT CTG TG-3'), reverse Richtung (5'-AAG CCT CTC CTT CCT CCT GT-3'). Insgesamt 2 µl cDNA-Lösung wurden während 35 Zyklen mit dem folgenden Programm amplifiziert: 1 min bei 94°C, 1 min bei 60°C (AR) und 67°C (β-Aktin) und 1 min bei 72°C. Die PCR-Produkte wurden mit Ethidiumbromid-Färbung sichtbar gemacht. β-Aktin diente als externer Standard.

### Nachweis der 5α-Reduktase und des Androgenrezeptors

In der Haut wird hauptsächlich das 5α-Reduktase Isoenzym-Typ-I exprimiert (T_{A} 65°C, Mg 15 mM, Produktlänge 369 bp). Die Androgenrezeptor-Expression läßt sich unter Verwendung eines Primerpaares von Exon 2 bis Exon 4 (T_{A} 60°C, Mg 25 mM, Produktlänge 269 bp) nachweisen.

Auf Proteinebene konnte der Androgenrezeptor mittels intrazellulärer Durchflußzytometrie (FACS) in SZ95-Sebozyten und Keratinozyten nachgewiesen werden. Die Färbung mit dem Antikörper muß intrazellulär erfolgen, da der Androgenrezeptor im Zellkern lokalisiert ist. Der Androgenrezeptor konnte auch mittels Westernblot in nativen SZ95-Sebozyten und Keratinozyten detektiert werden. Nach einer vierstündigen Transfektion der Sebozyten mit DOTAP separat als auch kombiniert mit einem Mismatch-Oligonukleotid (5'-CTG CCC TTC AAT CCC TGA GTT TGG C-3') sowie einer anschließenden Erholungsphase der Zellen von mindestens 24 Stunden war die Proteinexpression des Androgenrezeptors vergleichbar mit der in nicht transfizierten Zellen. Mit diesem Resultat kann eine veränderte (verminderte) Expression des Androgenrezeptors auf eine spezifische Antisense-Oligonukleotid/mRNA-Wechselwirkung zurückgeführt werden.

### Beispiel 2

### Untersuchung der Androgenrezeptor-Expression durch Immunoblotting

In Gegenwart des entsprechenden Transfektionsreagens (DOTAP für SZ95-Sebozyten and Poly-L-ornithin für Keratinozyten) wurden SZ95-Sebozyten und Keratinozyten 4 h mit unterschiedlichen AR-Antisense-Oligonukleotiden transfiziert. Die Konzentration der untersuchten Oligonukleotide betrug 0.1, 0.4 and 1.0 µM. Die Inhibierungsexperimente wurden in 6-Well Platten durchgeführt. Nach Transfektion wurden die Zellen in ihrem Kulturmedium inkubiert, um sich 14 h, 24 h, 48 h und 72 zu erholen. Schließlich wurden die Zellen in dem Lysis-Puffer M-PER (Pierce) für die Proteinextraktion suspendiert. Die Proteingesamtmenge wurde durch das Bradford-Verfahren bestimmt. 20 µg Gesamtprotein wurden mit 5-fachem Probepuffer und Reduktionsmittel vermischt, 10 Minuten auf 70°C erwärmt, und in einem 3-8%-igen Tris-acetat Gradientengel aufgetrennt. Die Proteine wurden auf eine Immobilon-PVDF Membran übertragen. Die Blots wurden über Nacht mit 0.25% Casein bei 4°C blockiert und dann bei Raumtemperatur 1 Stunde inkubiert, 1/1000 mit polyklonalem anti-AR Antikörper aus dem Hasen (Santa Cruz) 30 min verdünnt, und schließlich 1/10000 mit gegen Hase gerichtetem Immunoglobulin G, konjugiert an sekundäre Meerrettichperoxidase (Jackson Immuno Research) verdünnt. Die Blots wurden mit dem Chemielumineszenz-Verstärkungssystem (enhanced chemiluminescence) von Amersham ausgewertet. Die Intensität der AR-Proteinbanden wurde densitometrisch mit dem Computerprogramm TINA 2.0 bestimmt.

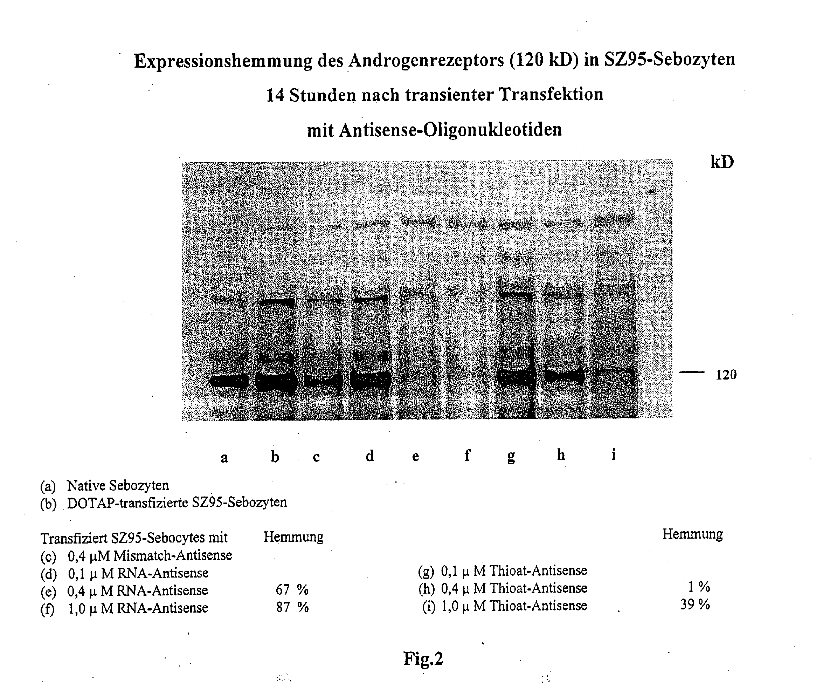

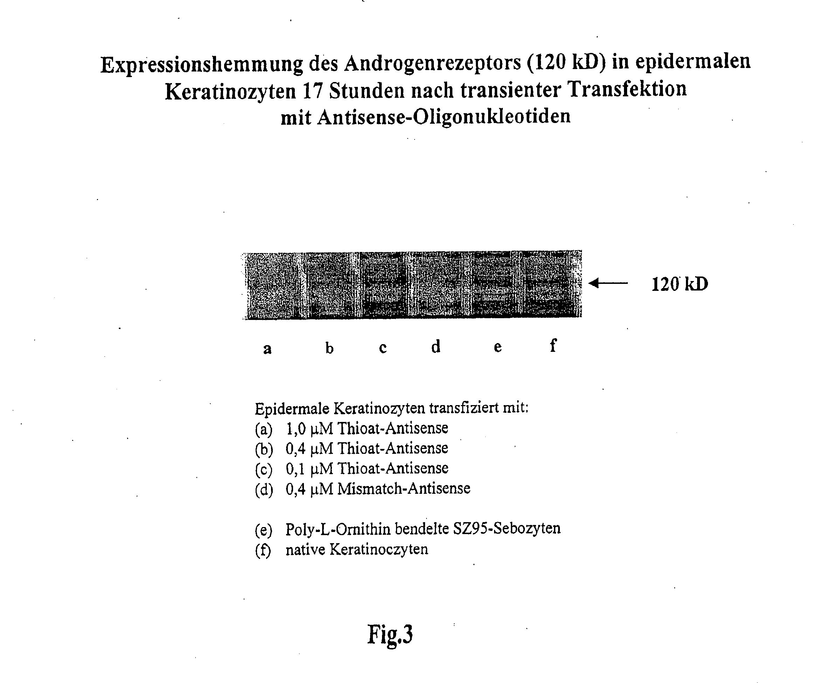

Die transiente Transfektion von SZ95-Sebozyten mit Thioat-(0,4 und 1,0 µM) und 2'-Methylribosyl-Antisense-Oligonukleotid (0,4 und 1,0 µM) ergab eine verringerte Proteinexpression des Androgenrezeptors nach 24 h (Fig.1). Nach längerer Erholungszeit, erreichte die Proteinexpression des Androgenrezeptors in transfizierten SZ95-Sebozyten wieder das Niveau der nativen Androgenrezeptor-Expression. Die erfolgreichste transiente Kontrolle der Androgenrezeptor-Expression wurde nach 14 h mit 1,0 µM 2'-Methylribosyl-Antisense-Oligonukleotid nachgewiesen, wobei sich eine Inhibierung von 87% der Androgenrezeptor-Expression im Vergleich zu nativen SZ95-Sebozyten ergab. Thioat-Antisense-Oligonukleotid (1,0 µM) ergab eine Abnahme der Androgenrezeptor-Expression von 39% (Fig.2). Diese Daten der transienten Transfektion von Epithelial-Keratinozyten zeigte nach 17 h Erholungszeit eine Reduktion der Androgenrezeptor-Expression von etwa 25% im Vergleich zu nativen Keratinozyten. Schon nach 24 h erreichte das Androgenrezeptor-Niveau der behandelten Keratinozyten wieder das Niveau der nativen Keratinozyten (Fig.3).

### Beispiel 3

Es wurde die biologische Aktivität des Androgenrezeptors in nativen und in mit Androgenrezeptor-Antisense-Oligonukleotid transfizierten SZ95-Sebozyten nach deren Stimulierung mit Androgenen untersucht. SZ95-Zellen wurden mit Testosteron und DHT bei Konzentrationen im Bereich von 1 µM and 0,01 pM inkubiert. Proliferierende Effekts wurden mit dem Kristallviolett-Assay gemessen. Mit diesem Test, durchgeführt in 24-Well-Platten, können lebende Zellen durch Färbung mit dem Kristallviolett-Farbstoff erkannt werden. Die Färbeintensität korreliert mit der Anzahl der lebenden Zellen.

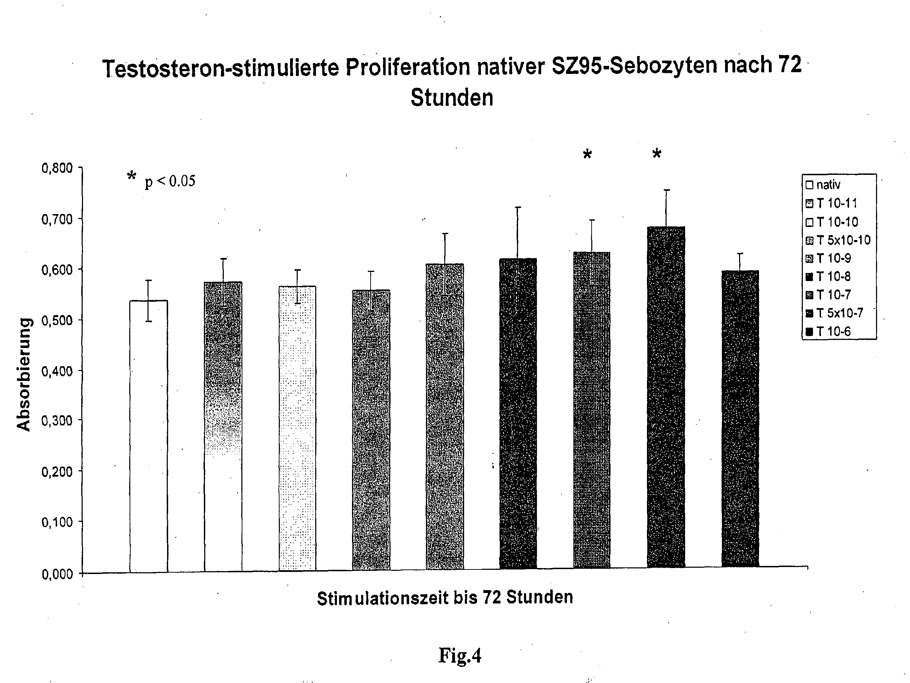

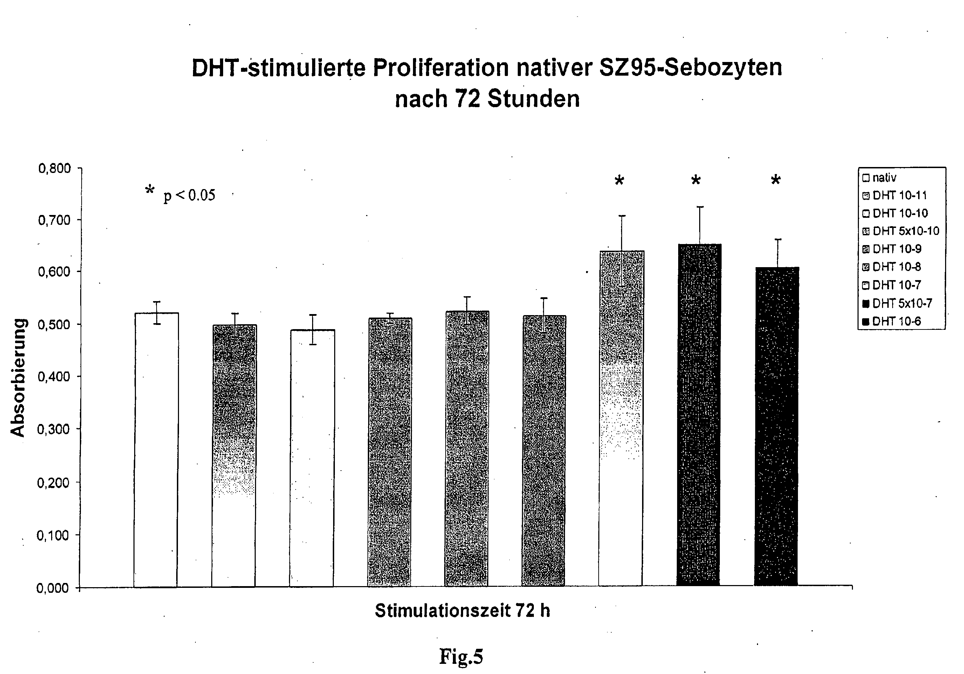

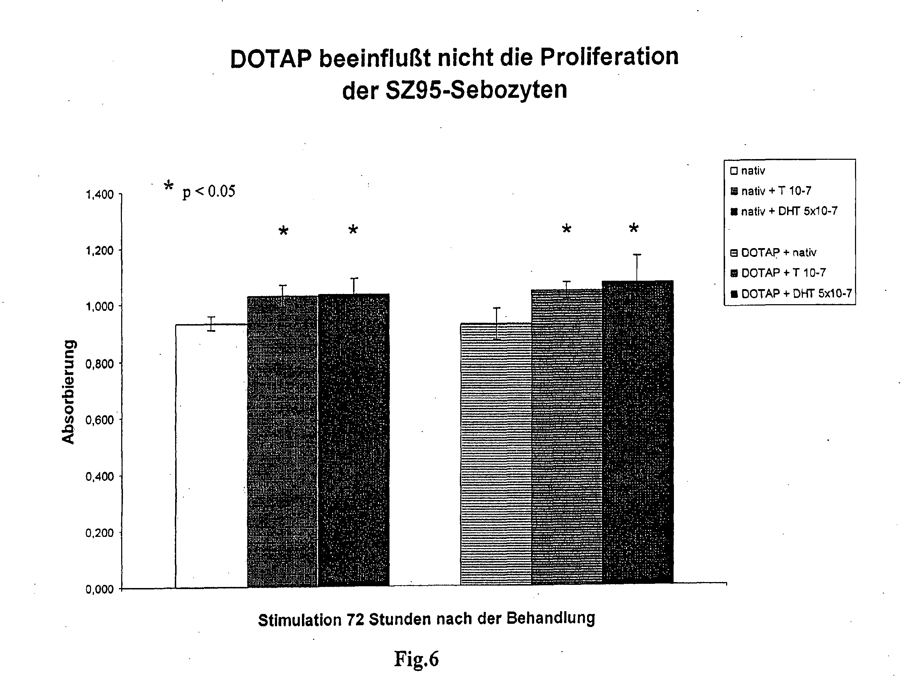

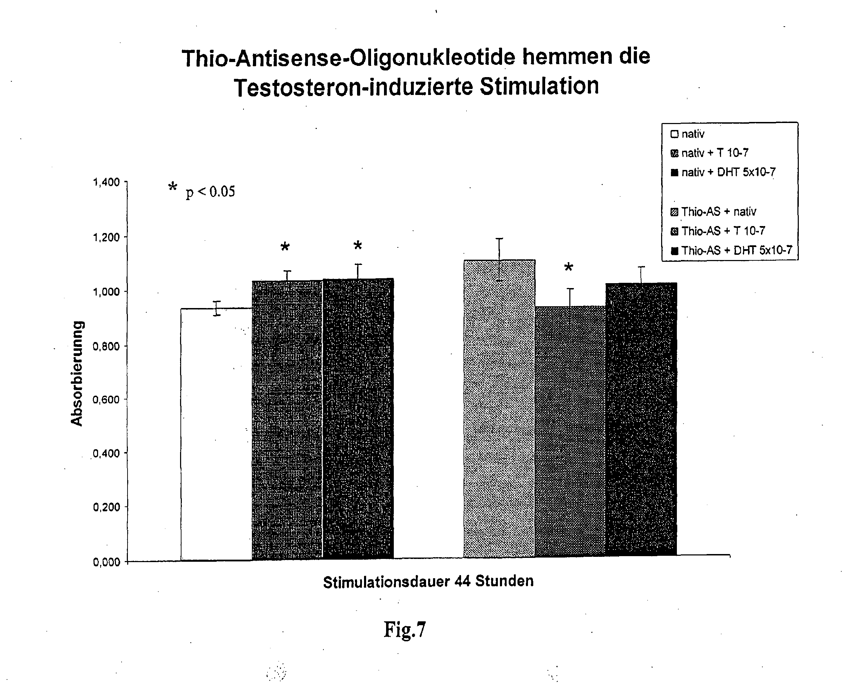

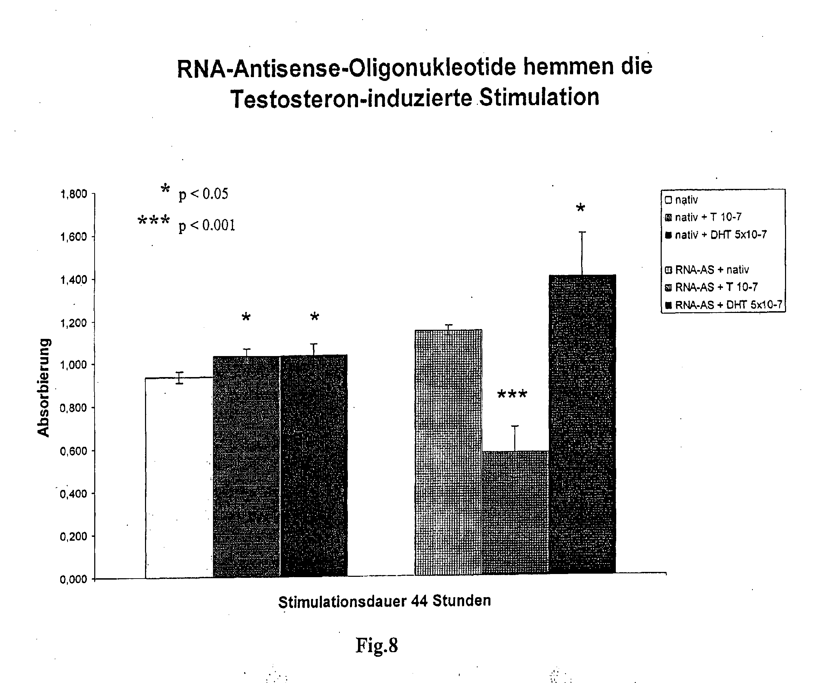

Sexualandrogene stimulierten die SZ95-Sebozyten-Proliferation nach 48 h. Nach 72 h war die Erhöhung der Proliferation bei Konzentrationen von 10⁻⁷ and 5×10⁻⁷ M Testosteron (Fig.4) und 10⁻⁷-10⁻⁶ M DHT (Fig. 5) signifikant. Es wurden transiente transfizierte SZ95-Sebozyten mit nativen Zellen verglichen, um den biologischen Effekt der zwei unterschiedlichen AR-Antisense-Oligonukleotide nach Androgenstimulierung zu bestimmen. SZ95-Sebozyten wurden 4 h mit 1 µM des beschriebenen Phosphorothioat-Oligonukleotids oder 1 µM des beschriebenen Methylribosyl-Oligonukleotids transfiziert. Nach einer Erholungszeit von 14 h wurden die SZ95-Sebozyten mit 10⁻⁷ M Testosteron oder 5×10⁻⁷ M DHT inkubiert. Die Kontrolle, lediglich mit Tranfektionsreagens DOTAP behandelte SZ95-Sebozyten, zeigten ähnliche Proliferationraten wie native SZ95-Sebozyten (Fig. 6). Andererseits inhibierten die Thioat-Antisense-Oligonukleotide die Testosteron-induzierte Stimulation der SZ95-Sebozytenproliferation (Fig. 7). Die mit dem beschriebenen Androgenrezeptor-Thioat-Antisense-Oligonukleotid transfizierten SZ95-Sebozyten zeigten das gleiche Proliferationsmuster wie native Zellen in der Gegenwart von Testosteron. Die Inhibierung der Testosteroninduzierten Stimulation der Zellproliferation durch RNA-Oligonukleotide war hoch signifikant and wohl toxisch (Apoptose/Nekrose) für SZ95-Sebozyten (Fig. 8).

So konnte auf Grundlage der vorstehend beschriebenen Beispiele nachgewiesen werden, dass die erfindungsgemäß verwendeten molekular hergestellten Moleküle (Antisense-Oligonukleotide, Ribozyme, stabilisierte Ribozyme, Aptamere, Spiegelaptamere, chimerische RNA/DNA-Oligonukleotide, nackte Plasmid-DNA, oder liposomal verkapselte DNA, insbesondere die Antisense-Oligonukleotide hoch-effizient in den humanen Zielzellen eine spezifische Inhibierung der hormonellinduzierten, insbesondere der Androgen-induzierten Stimulation der Sebozyten- bzw. Keratinozyten-Proliferation herbeiführen können und daher zur Untersuchung, und Therapie der Akne und der akneiformen Dermatosen, inkl. der Rosazea speziell geeignet und einsatzfähig sind.

## Patentansprüche

1. Verwendung von mindestens einem molekularbiologisch hergestelltem, nicht-viralen Wirkstoff mit Spezifität für eine Nukleinsäure-Zielsequenz eines Gens, das für das 5α-Reduktase-I-Isoenzym kodiert, ausgewählt aus Antisense-Oligonukleotiden, Ribozymen, stabilisierten Ribozymen, Aptameren, Spiegelaptameren oder chimären RNA/DNA-Oligonukleotiden, zur Herstellung eines Medikaments zur Behandlung oder Therapie der Akne und der akneiformen Dermatosen, inkl. der Rosazea.

2. Verwendung nach Anspruch 1, **dadurch gekennzeichnet, dass** das mindestens eine Antisense-Oligonukleotid komplementär zu einer Ribonukleinsäuresequenz ist, die von einer für das 5α-Reduktase-I-Isoenzym kodierenden Gensequenz stammt.

3. Verwendung nach Anspruch 2, **dadurch gekennzeichnet, dass** das Antisense-Oligonukleotid komplementär zu einer Einzelstrang-RNA oder Einzelstrang-DNA oder einer DNA innerhalb eines Duplexstranges ist.

4. Verwendung nach einem der Ansprüche 1 bis 3, **dadurch gekennzeichnet, dass** das Antisense-Oligonukleotid chemisch modifiziert ist.

5. Verwendung nach Anspruch 1, **dadurch gekennzeichnet, dass** die Nukleinsäure-Zielsequenz in Epithelzellen, insbesondere humanen Epithelzellen vorliegt.

6. Verwendung nach Anspruch 1, **dadurch gekennzeichnet, dass** die Nukleinsäure-Zielsequenz in Zellen der humanen Haut vorliegt.

7. Verwendung nach Anspruch 5 oder 6, **dadurch gekennzeichnet, dass** die Nukleinsäure-Zielsequenz in humanen Sebozyten und/oder Keratinozyten vorliegt.

8. Verwendung nach Anspruch 8, **dadurch gekennzeichnet, dass** die Zielzellen humane Sebozyten und/oder Keratinozyten sind.

9. Verwendung nach einem der vorhergehenden Ansprüche, **dadurch gekennzeichnet, dass** das mindestens eine Antisense-Oligonukleotid durch Liposomen-vermittelte Transfektion in die Zielzellen eingebracht wird.

10. Verwendung gemäß einem der vorangehenden Ansprüche, wobei das Antisense-Oligonukleotid mit mindestens einem weiteren, herkömmlichen Anti-Akne-Wirkstoff kombiniert wird.

11. Verwendung gemäß einem der vorangehenden Ansprüche, wobei eine orale und/oder topische Applikation erfolgt.

12. Verwendung gemäß einem der Ansprüche 9 und 10 zur Zubereitung einer pharmazeutischen Zusammensetzung mit mindestens einem der in den Ansprüchen 1 bis 8 angegebenen Merkmalen zusammen mit einem pharmazeutisch akzeptablen Träger und gegebenenfalls weiteren Hilfsstoffen.

13. Verwendung eines molekularbiologisch hergestellten, nicht-viralen Wirkstoffs mit Spezifität für eine Nukleinsäure-Zielsequenz eines Gens, das für das 5α-Reduktase-I-Isoenzym kodiert, ausgewählt aus Antisense-Oligonukleotiden, Ribozymen, stabilisierten Ribozymen, Aptameren, Spiegelaptameren oder chimären RNA/DNA-Oligonukleotiden, zur Untersuchung der Akne und der akneiformen Dermatosen, inkl. der Rosazea, mittels Modifizierung von Zellen in vitro.

## Claims

1. The use of at least one molecular-biologically prepared non-viral active agent having specificity for a target nucleic acid sequence of a gene, which codes for the 5α-reductase-I-isoenzyme, selected from antisense oligonucleotides, ribozymes, stabilized ribozymes, aptamers, mirror aptamers, or chimeric RNA/DNA oligonucleotides, for manufacturing a drug for the treatment or therapy of acne and of the acneiform dermatoses, including rosacea.

2. The use according to claim 1, **characterized in that** the at least one antisense oligonucleotide is complementary to a ribonucleic acid sequence, which originates from a gene sequence coding for the 5α-reductase-I-isoenzyme.

3. The use according to claim 2, **characterized in that** the antisense oligonucleotide is complementary to a single-stranded RNA or a single-stranded DNA or a DNA within a double-strand.

4. The use according to one of claims 1 to 3, **characterized in that** the antisense oligonucleotide is chemically modified.

5. The use according to claim 1, **characterized in that** the target nucleic acid sequence is present in epithelial cells, in particular human epithelial cells.

6. The use according to claim 1, **characterized in that** the target nucleic acid sequence is present in cells of the human skin.

7. The use according to claim 5 or 6, **characterized in that** the target nucleic acid sequence is present in human sebocytes and/or keratinocytes.

8. The use according to claim 8, **characterized in that** the target cells are human sebocytes and/or keratinocytes.

9. The use according to one of the preceding claims, **characterized in that** the at least one antisense oligonucleotide is introduced into the target cells by liposome-mediated transfection.

10. The use according to one of the preceding claims, wherein the antisense oligonucleotide is combined with at least one further conventional anti-acne active agent.

11. The use according to one of the preceding claims, wherein an oral and/or topical application is effected.

12. The use according to one of claims 9 and 10 for preparing a pharmaceutical composition having at least one of the features listed in claims 1 to 8 together with a pharmaceutically acceptable carrier and further adjuvants where appropriate.

13. The use of a molecular-biologically prepared non-viral active agent having specificity for a target nucleic acid sequence of a gene, which codes for the 5α-reductase-I-isoenzyme, selected from antisense oligonucleotides, ribozymes, stabilized ribozymes, aptamers, mirror aptamers, or chimeric RNA/DNA oligonucleotides, for examining acne and the acneiform dermatoses, including rosacea, by modifying cells in vitro.

## Revendications

1. Utilisation d'au moins un agent actif non viral produit par un procédé biologique moléculaire et ayant une spécificité pour une séquence cible d'un acide nucléique d'un gène, qui code pour la 5α-réductase-I-isoenzyme, choisi à partir d'oligonucléotides antisens, ribozymes, ribozymes stabilisées, aptamères, aptamères miroirs ou oligonucléotides ARN/ADN chimériques, pour la production d'un médicament pour le traitement ou la thérapie de l'acné et des dermatoses acnéiformes, inclusivement la rosacée.

2. Utilisation selon la revendication 1, **caractérisée en ce que** l'au moins un oligonucléotide antisens est complémentaire à une séquence d'acide ribonucléique, qui provient d'une séquence génétique codant pour la 5α-réductase-I-isoenzyme.

3. Utilisation selon la revendication 2, **caractérisée en ce que** l'oligonucléotide antisens est complémentaire à un ARN simple brin ou ADN simple brin ou un ADN au-dedans d'un double brin.

4. Utilisation selon une des revendications 1 à 3, **caractérisée en ce que** l'oligonucléotide antisens est chimiquement modifié.

5. Utilisation selon la revendication 1, **caractérisée en ce que** la séquence cible d'un acide nucléique se trouve dans des cellules épithéliales, en particulier cellules épithéliales humaines.

6. Utilisation selon la revendication 1, **caractérisée en ce que** la séquence cible d'un acide nucléique se trouve dans des cellules de la peau humaine.

7. Utilisation selon la revendication 5 ou 6, **caractérisée en ce que** la séquence cible d'un acide nucléique se trouve dans des sébocytes et/ou kératinocytes humains.

8. Utilisation selon la revendication 8, **caractérisée en ce que** les cellules cibles sont des sébocytes et/ou kératinocytes humains.

9. Utilisation selon une des revendications précédentes, **caractérisée en ce que** l'au moins un oligonucléotide antisens est introduit dans les cellules cibles par transfection médiée par liposomes.

10. Utilisation selon une des revendications précédentes, dans laquelle l'oligonucléotide antisens est combiné avec au moins un agent actif anti-acné conventionnel additionnel.

11. Utilisation selon une des revendications précédentes, dans laquelle une application orale et/ou topicale est effectuée.

12. Utilisation selon une des revendications 9 et 10 pour la préparation d'une composition pharmaceutique ayant au moins une des caractéristiques mentionnées dans les revendications 1 à 8 ensemble avec un porteur pharmaceutiquement acceptable et le cas échéant d'autres adjuvants.

13. Utilisation d'un agent actif non viral produit par un procédé biologique moléculaire et ayant une spécificité pour un séquence cible d'un acide nucléique d'un gène, qui code pour la 5α-réductase-I-isoenzyme, choisi à partir d'oligonucléotides antisens, ribozymes, ribozymes stabilisées, aptamères, aptamères miroirs ou oligonucléotides ARN/ADN chimériques, pour l'examen de l'acné et des dermatoses acnéiformes, inclusivement la rosacée, par modification de cellules in vitro.
